# EUROPEAN PATENT APPLICATION

(11) **EP 4 575 586 A1**
(43) Date of publication of application: **25.06.2025**
(21) Application number: 24221422.9
(22) Date of filing: 19.12.2024
(51) Int. Cl.: G01T 1/17, G01T 1/29

(54) **PET SYSTEM INCLUDING MULTIPLEXED OR MUXED READOUT BOARDS**

(30) Priority: 19.12.2023 US 202363612094 P
(71) Applicant: Cubresa Inc., Winnipeg, Manitoba R3B 0P4 (CA)
(72) Inventor: Schellenberg, James, Winnipeg, R3B 0P4 (CA)
(74) Representative: Franke, Dirk

(57) **Abstract**

Described herein is a reduced PET design that uses shared and multiplexed Analog to Digital Converter (ADC) resources in which changes to the electronic cabinet are made to allow multiplexing. Specifically, individual cables from regions of detector blocks that geometrically cannot be in coincidence with each other are muxed together at the ADC chip.

## Description

### PRIOR APPLICATION INFORMATION

The instant application claims the benefit of US Provisional Patent Application 63/612,094, filed December 19, 2023, entitled "PET SYSTEM INCLUDING MULTIPLEXED OR MUXED READOUT BOARDS", the entire contents of which are hereby incorporated by reference for all purposes.

### BACKGROUND OF THE INVENTION

Positron Emission Tomography (PET) systems today are designed with dedicated analog to digital (ADC) systems that allow for the maximum in performance but unfortunately also with the maximum in cost, size and heat. These previous PET systems stood apart from associated CT or MRI systems and so maximum size designs were acceptable. In the field of PET-MRI combined systems, however, maximum size designs may not be the best approach.

PET medical imaging systems are typically arranged with a multitude of scintillation elements and readout boards organized around the object to be imaged. A PET system coincidence occurs when two scintillations occur at the same time, which provides a line of response along which the annihilation event must have occurred. These annihilation events occur inside the item being imaged.

For scintillator elements arranged around a body, some coincidence geometries are impossible, such as coincidence pairs that define a line of response that does not go through the body being imaged.

Multiplexing is commonly discussed within PET readout methods. Multiplexing is a way of reducing the number of cables that come out of the scintillator block and which in turn leads to channel count reduction and cost reduction. This type of multiplexing refers to using resistive readout, capacitive readout, or hybrid readout methods with an array of pixels. This multiplexing occurs at the level of one block and is called intrablock muxing.

More unique methods to perform multiplexing have been discussed in US Patent 9,903,961 by Ng et al. In this approach, multiplexing is applied to the row and column organization of the pixels. However, this is still a multiplexing at the intrablock level.

Multiplexing of the fast outputs of the pixels is also known in the art and is used to reduce the number of signals that need to be processed.

Examples of intrablock multiplexing include the publications and references within: Joshua W. Cates, Matthew F. Bieniosek, Craig S. Levin, "Highly multiplexed signal readout for a time-of flight positron emission tomography detector based on silicon photomultipliers," J. Med. Imag. 4(1), 011012 (2017), doi: 10.1117/1.JMI.4.1.011012 and " ELECTRONIC READOUT STRATEGIES FOR SILICON PHOTOMULTIPLIER-BASED POSITRON EMISSION TOMOGRAPHY DETECTORS", Stanford Thesis, Matthew Bieniosek, March 2016. Also as prior art is Schellenberg "Readout Board Muxing for PET Systems". Filed Sept 21, 2020, US 2021/0088682 A1, the entire contents of which are incorporated herein by reference for all purposes, particularly for the teachings regarding muxing arrangements for PET systems.

### SUMMARY OF THE INVENTION

According to a first aspect of the invention, there is provided a positron emission tomography imaging system comprising:
a plurality of slow output cables, each slow output cable having a detector end and at least a first chip end;
a plurality of detector blocks, each respective corner of each one of the plurality of the detector blocks being connected to the detector end of a respective one of the plurality of slow output cables;
at least a first respective one detector block and a second respective one detector block being multiplexed together by connecting the first chip end of the slow output cable connected to the first respective one detector block and the first chip end of the slow output cable connected to the second respective one detector block to one analog-to-digital converter chip.

### BRIEF DESCRIPTION OF THE DRAWINGS

Figure 1 shows a common BrainPET system design
Figure 2 is a schematic diagram of Detector Block to ADC wiring.
Figure 3 shows Detector Blocks connected to ADC circuits in a standard 1 to 1 design approach.
Figure 4 shows a detector block with slow outputs connected to each corner of the detector block.
Figure 5 shows a multiplexing connection between Detector Blocks and ADC circuits.
Figure 6 shows a first method of multiplexing the slow and fast signals.
Figure 7 shows a second method of multiplexing the slow and fast signals.
Figure 8 shows a third method of multiplexing the slow signals when fast signals are not available.
Figure 9 shows an example of four slow signal voltages over time and the sum of the four slow signal voltages.

### DESCRIPTION OF THE PREFERRED EMBODIMENTS

Unless defined otherwise, all technical and scientific terms used herein have the same meaning as commonly understood by one of ordinary skill in the art to which the invention belongs. Although any methods and materials similar or equivalent to those described herein can be used in the practice or testing of the present invention, the preferred methods and materials are now described. All publications mentioned hereunder are incorporated herein by reference.

Described herein is an interblock multiplexing technique for use for example with PET inserts that integrate with MRI systems. As discussed herein, maximum designs may not be the best approach. We introduce reduced PET design approaches that use shared and multiplexed Analog to Digital Converter (ADC) resources. Whereas the previous Schellenberg patent application US 2021/0088682 A1 discussed changes to the readout boards (also called Block Carrier Boards in this patent) to allow multiplexing, described herein are methods wherein changes are made to the electronic cabinet design and components to allow multiplexing. An important difference between these two techniques is that the earlier Schellenberg patent application required changes to the insert hardware, whereas the methods described below require changes only to the electronic cabinet hardware, which allows for an easier upgrade path for customers.

According to an aspect of the invention, there is provided a positron emission tomography imaging system comprising:
a plurality of slow output cables, each slow output cable having a detector end and at least a first chip end;
a plurality of detector blocks, each respective corner of each one of the plurality of the detector blocks being connected to the detector end of a respective one of the plurality of slow output cables;
at least a first respective one detector block and a second respective one detector block being multiplexed together by connecting the first chip end of the slow output cable connected to the first respective one detector block and the first chip end of the slow output cable connected to the second respective one detector block to one analog-to-digital converter chip.

In some embodiments, the first respective one detector block and the second respective one detector block are geometrically positioned within the positron emission tomography imaging system such that the first respective one detector block and the second respective one detector block cannot be in coincidence. For example, if the PET imaging system is a cylindrical system with detector blocks organized on the cylinder, the only location where scintillation events can actually occur from the object under study will be inside the cylinder. It is therefore not possible for two adjacent detector blocks to have any object between them. Further, if one considers a set of four detector blocks, all of which are located along a given axial direction, it is not possible for the object under study to be between these detector blocks. This means that as long as the amount of radioactivity that is being observed is quite low, this set of blocks can be reasonably expected to not have events at the same time.

One source of potential error in this approach concerns the use of LYSO scintillator in the detector blocks. LYSO as a scintillation material is itself radioactive, and generates noise in the image due to this characteristic. The amount of radioactivity generated by a block of LSYO depends on its size. LYSO material generates 39 counts per second per gram of material and weighs 7.1 grams per cubic cm. For a LYSO block of 2 cm height with 5 cm width and 4 cm length, equal to 40 cubic cm, there will be 7.1 × 40 × 39 = 10.9 Kcps of background noise. The energy emissions of LYSO fall outside of the 511 keV bands that are of interest with PET, and so not all of this background noise ends up affecting the image. If this noise overlaps in time with an actual scintillation event caused by the object under test, the voltage levels that are measured can be affected. Depending on the size of the detector blocks, this background noise may limit the amount of multiplexing that can be done using the methods outlined within.

In some embodiments, the positron emission tomography imaging system further comprises a plurality of fast output cables, each respective one fast output cable connected at a detector end to a respective one detector block and at a first chip end to a timing chip.

In some embodiments, each fast output cable has a second chip end connected to a block selection chip.

In some embodiments, each slow output cable has a second chip end for connecting each corner of a given detector block to one analog-to-digital converter chip, one timing chip or one block selection chip.

In some embodiments, each slow output cable has a third chip end for connecting each corner of a given detector block to a timing chip.

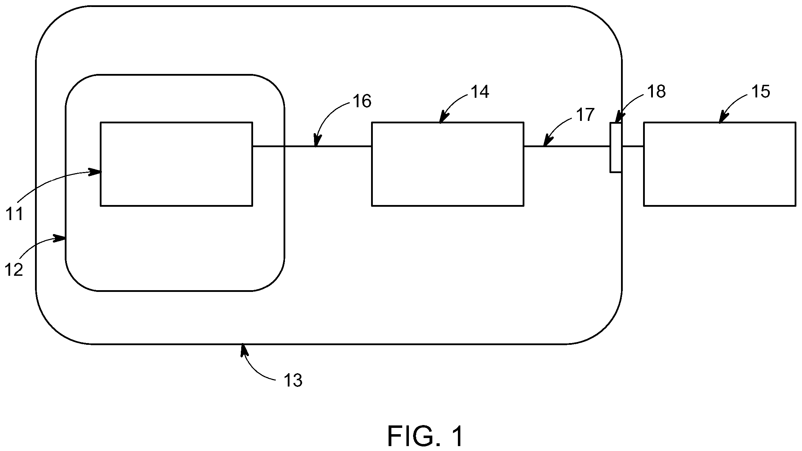

Figure 1 shows a common system design. Inside an MRI shielded room 13 is an MRI System 12. Into the MRI System 12 is placed a BrainPET insert 11 which is designed to work simultaneously with the MRI System 12. The BrainPET Insert 11 is connected to an electronics cabinet 14 which is also in the MR shielded room 13. The electronics cabinet 14 contains amplifier boards and analog to digital conversion (ADC) circuits. The electronics cabinet is connected by fiber 17 through the filter panel 18 to the workstation 15 which processes the digital information and shows the images on a screen. A copper line 16 connects the BrainPET insert 11 to the Electronics Cabinet 14. Power for the BrainPET insert 11 is provided from the Electronics Cabinet 14, which is plugged into a suitable outlet on the wall of the MRI shielded room 13.

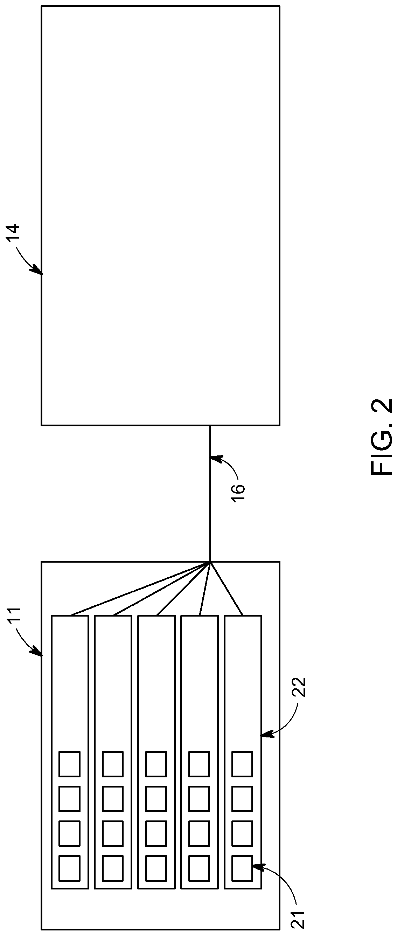

Figure 2 is a schematic diagram of Block to ADC wiring. Inside the BrainPET Insert 11 are a number of Detector Blocks 21. The Detector Blocks 21 consist of a scintillator and a SiPM board, held together using methods known in the art. The Detector Blocks 21 are attached to a Block Carrier Board 22. The Block Carrier Board is aligned axially along the BrainPET Insert 11. There are 4 Detector Blocks 21 per Block Carrier Board 22. As an example, if each Detector Block 21 is 50 mm in axial length, with a gap of 2 mm between Detector Blocks 21, which would then give a total field of view in the Axial direction of the BrainPET system of 4x 50 + 3 x 2 = 206 mm. The number of Block Detector Boards 22 and the width of the Block Detector Boards 22 will determine the circumference of the cylindrical and diameter of the BrainPET insert 11. Typically the Block Detector Boards 22 are positioned as close as possible to one another to optimize the probability that the scintillators in the Detector Blocks 21 will intercept the radiation from inside the object under test. For example, if there are 16 Block Detector Boards 22 and if the Block Detector Boards are 60 mm in width and if a 2 mm gap is allowed between the Block Detector Boards 22, then the circumference will be 60 * 16 + 2 * 16 = 992 mm. This gives a diameter of 315 mm. The BrainPET Insert 11 is connected to the Electronics Cabinet 14 by the copper cable 16.

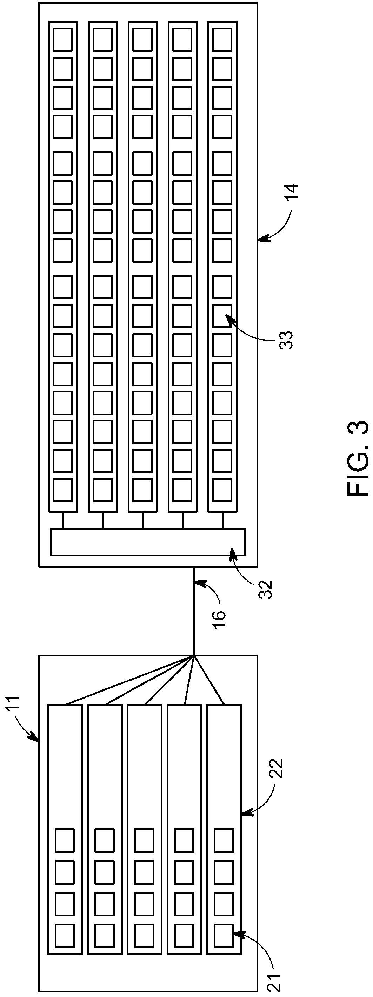

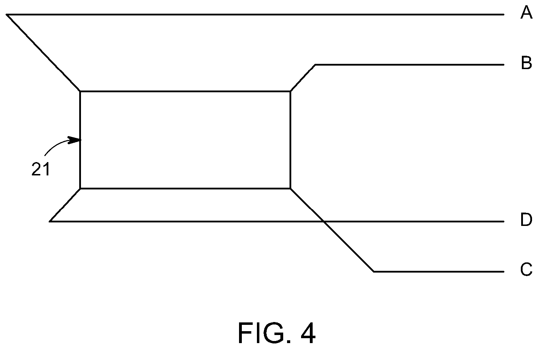

Figure 3 shows Detector Blocks 21 on Block Carrier Boards 22 connected by copper cable 16 to the circuits inside the Electronics cabinet 14, these circuits including an amplifier board 32 and ADC circuits 33. In this diagram it is assumed that a standard approach is used without interblock multiplexing. The standard approach is one ADC circuit 33 required for each output line from the detector block 21. In this design each detector block outputs 4 slow signals as shown in Figure 4. Each slow signal needs to terminate in an ADC circuit 33, which means there are 4 ADC Circuits 33 for each Detector Block 21. This leads to a cost problem for these systems, because in order to increase the count rate of a PET system it is sometimes necessary to reduce the size of the Detector Block 21. For example, if one assumes a BrainPET system that is intended to have an axial field of view of 200 mm and a diameter of approximately 315 mm, this requires approximately 64 Detector Blocks 21 of size 50 mm by 50 mm. These 64 Detector Blocks 21 require x * 64 = 256 ADC circuits for termination in a standard design. If one wishes to increase the count rate of the system by reducing the size of the Detector Blocks 21 one still needs to cover the same size of diameter and axial extent, because the human head is a fixed size. If the number of Detector Blocks 21 are increased to 256 instead of 64, the number of ADC circuits 33 increase by the same factor because each Detector Block 21 still needs four slow voltage output lines regardless of the size of the block. Therefore 256 detector blocks 21 require 1024 ADC circuits 33.

Figure 4 shows a detector block 21 with slow outputs A, B, C, and D connected from each corner of the detector block. These corner outputs are created from the resistive and capacitive network connected to the SiPM detectors in a manner known in the art. There may be also a fast output line being output from the Detector Block 21, but this fast line is not shown in this figure.

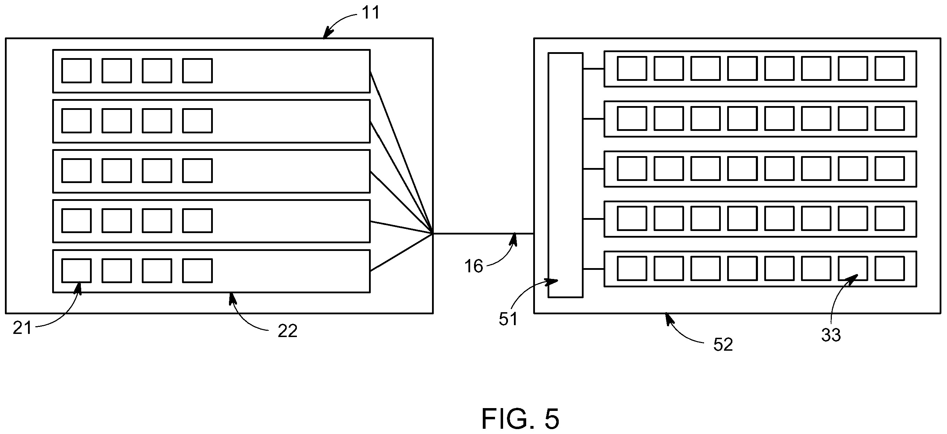

Figure 5 shows a 2 to 1 interblock multiplexing connection between Detector Blocks 21 and ADC circuits 33. In this figure is shown 5 Block Carrier Boards 22 with each board having 4 Detector Blocks 21. Each detector block 21 outputs 4 slow signals, leading to a total of 4 * 4 * 5 = 80 slow output lines within the copper cable 16. Normally this would require 80 ADC circuits 33. In the multiplexing electronics cabinet 52 is a multiplexing amplifier board 51 and an ADC circuit 33. The multiplexing circuit design occurs within the new amplifier board 51. The number of ADC circuits 33 required is 50% reduced to 40 ADC circuits instead of the standard 80. There are several methods to multiplex these signals.

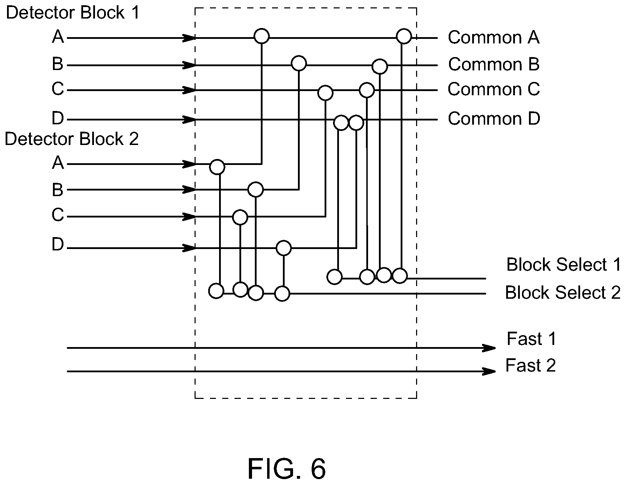

Figure 6 shows a first method of multiplexing the slow and fast signals. This multiplexing occurs in the amplifier board. The A, B, C, D signals from Detector Block 1 are joined together with the A, B, C, D signals from Detector Block 2 using a common connection. This common connection is used to create a common A, common B, common C and common D input to 4 ADC circuits which are not shown. The four signals from Detector Block 1 are jointly connected to a block select line 1. The four signals from Detector Block 2 are jointly connected to a block select line 2. Timing comes from connecting the fast output signals fast 1 and fast 2 into 2 separate timing circuits. In this multiplexing approach, the energy and x, y position within a detector block are determined from the common A, common B, common C and common D signal lines. The block select signals are used to determine in which of the 2 blocks the scintillation event occurred. In this figure there are 10 input signals (2 fast and 8 slow).

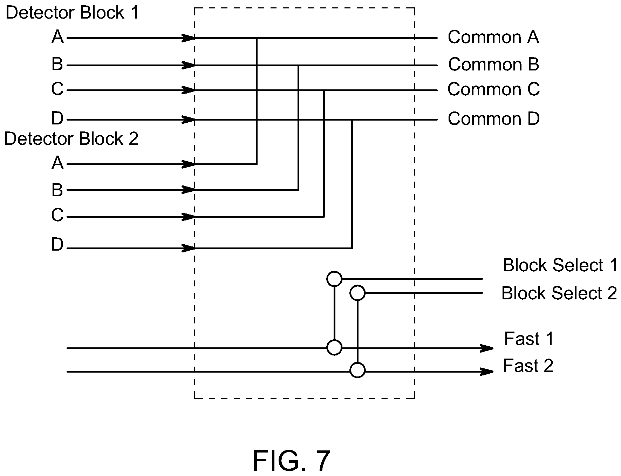

Figure 7 shows a second method of multiplexing the slow and fast signals. In this embodiment, fast output lines are used for both block selection and event timing. This method also uses 4 ADC circuits to terminate the common A, common B, common C and common D signal lines. The A, B, C, D signal lines from detector block 1 and 2 are connected together in the same manner as shown in Figure 6. In this example the energy and position performance may be better than in the previous figure because the slow signal lines A, B, C, D are not loaded down with the additional need to do block select. This might be a small voltage loading and buffering methods known in the art can be used to connect these various signal lines. In this figure there are 10 input signals (2 fast and 8 slow).

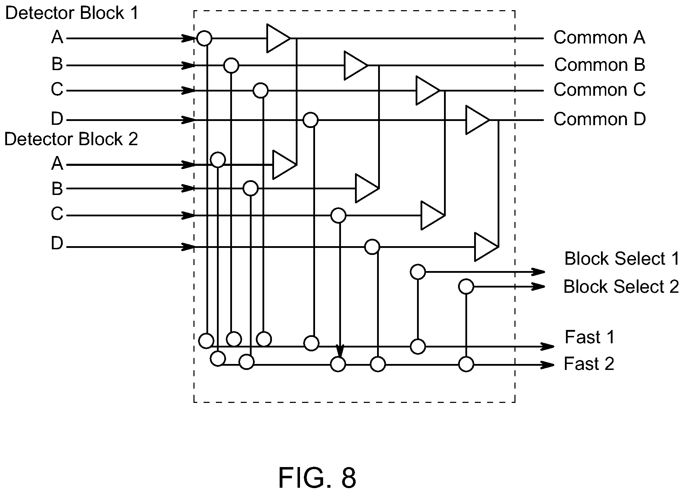

Figure 8 is a schematic diagram of a third embodiment of an amplifier board wiring arrangement suitable for interblock multiplexing. In this embodiment, the slow lines are used for all four functions of energy, position, event timing and block select. This can be done if there are appropriate isolating circuits to allow the multiple functions. Buffer amplifiers are shown on the upper slow lines but circuitry for creating fast outputs from the slow outputs is known in the art and is not shown. In this figure there are 8 input signals (0 fast and 8 slow).

For this interblock muxing method, one question is whether overlapping events might occur that causes error in the energy, position, timing and block select functions. Currently there is a large trend within medical imaging in which Artificial Intelligence (Al) methods are used to reduce the dose amount required to achieve a given image quality. If these Al methods are successful it means that not as much radioactivity is required within the imaging volume and this leads to a reduction in the number of events that will occur.

For example, assume that 20 detector blocks are arranged around a human head for PET imaging purposes. The 20 blocks are typically not very busy dealing with scintillation events, with, for example a 6 MBq human head positioned in the middle of the N blocks creating an estimated 6 MBq / 20 = 300 kcps per block.

If the scintillator, SiPM, resistor and capacitor network, copper cable, amplifier board, ADC circuits and associated electronics leads to an event timespan of 0.1 microsecond, the 300 kcps per block leads to an approximate busy percentage of 3% of the time. This estimate does not include detailed arrival process calculations. This means that 97% of the time the block and the associated slow signal lines and the ADC electronics circuits are not active with an event. For a system that is designed using standard brute force methods, each output line would be coupled 1:1, with 1 ADC block associated with 1 slow signal line. This means that each ADC system is only operating 3% of the time. There is a more efficient way to deal with the ADC design and we propose multiplexing methods so that each ADC can be more optimally used while allowing easy upgrade of the system using changes to the electronic cabinet.

For example, at these scintillation event levels, it is possible to use the same ADC system for four blocks at the same time and there would be little overlap of events. More specifically, depending on the exact design of the scintillator block geometry, it is possible to couple together blocks that are known a priori to not be able to have a scintillation event at the same time because of their locations relative to what is being imaged. If one assumes that a radioactive object completely fills the inside of the cylindrical PET system, it is still impossible to have scintillation events occurring between detector blocks that are along the same axial direction.

As known by those of skill in the art, the faster the scintillator is read, the greater the amount of multiplexing is possible. Faster electronics also leads to higher allowed level of multiplexing.

The multiplexing methods discussed here only pertain to the slow signal outputs from the corners of the detector blocks. The fast outputs that can be used for timing are not affected.

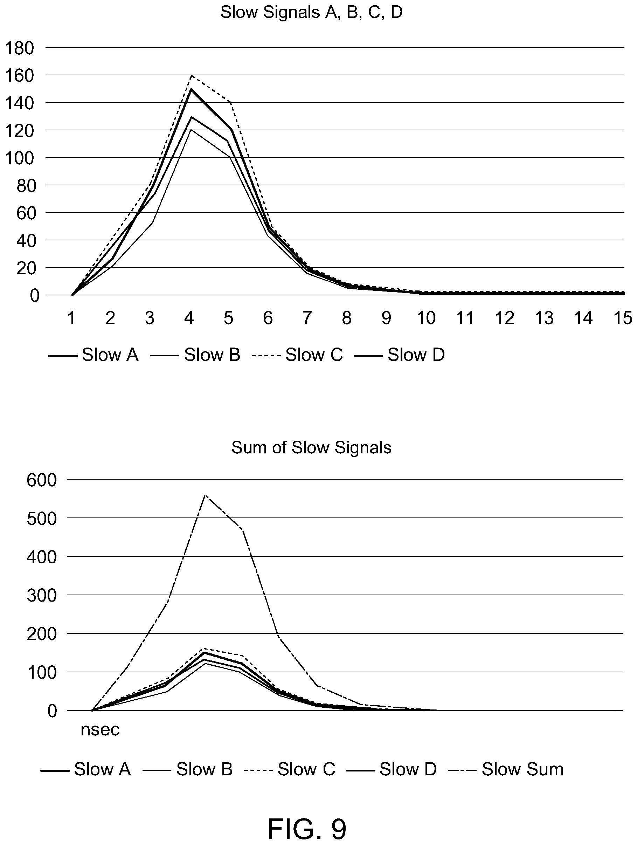

In Figures 6, 7 and 8 the input signal lines A, B, C, and D are connected together. In Figure 9 is shown the result of this connection. The voltages add together. The 4 slow pulses added together have a sharper rise time and a more pronounced peak voltage, which aids in the identification of the pulse timing.

The choice between which approach to use affects the timing accuracy, the type of insert that is used, the size of the cabling between insert and electronics cabinet and the energy resolution performance. The different methods may also affect the spatial accuracy of the PET system, because the slow signals are used to generate the x,y coordinates of the scintillation event, and if the slow signal voltage accuracies are affected by loading the cable, this may alter the x,y reading. In the example of Figure 6, there needs to be 4 slow lines and 1 fast line coming from each block. In Figure 7, this is also needed. In Figure 8, however, the insert only needs to output the slow lines in order to allow the method to work. In all cases, the (fast and) slow lines are analog lines.

The block select lines terminate in relatively simple electronic circuits, which is why there is a cost advantage to using this approach. The block select electronics are much cheaper than the ADC electronics. The circuits also use less power, generate less heat and require less room.

The example above used a multiplexing reduction of 2 to 1. If instead we use a 4 to 1 multiplexing reduction, the effect of the multiplexing on cost and size may be better appreciated. In this case, the number of input lines to the amplifier board will be 4 slow lines from each block and 1 fast line from each block, which equates to 16 slow lines and 4 fast lines. In this case, Figure 6 would be modified to have outputs of 4 slow lines, 4 fast lines and 4 block select lines. Figure 7 would be modified to have the same outputs, with the block select created from the fast lines. Figure 8, however, would be modified to have amplifier board inputs of 16 slow lines and outputs of 4 slow lines, 2 fast lines for timing and 4 block select lines. In all 3 examples, there would need to be 4 ADCs to terminate the slow lines.

In some embodiments, the fast output lines are terminated in timing circuits.

As will be appreciated by one of skill in the art, for the slow signals, you need ADC termination because you want to know the voltage as accurately as possible. In the case of the block select, you instead just want to see the transition. That is, if the circuit is triggered, you know that the block is selected.

In the case of timing, the circuits are more complicated and specialized and the methods are known in the art.

The block select lines are generated from the slow outputs using a wired AND combination of all 4 lines per block. Notice that in contrast to the earlier Schellenberg patent application, the block select lines are generated here from the slow lines and are generated within the amplifier board, not near the readout board circuits inside the insert. All three of the methods discussed here can be done within the electronic cabinet and not in the insert.

The advantage of doing this multiplexing at the amplifier board is that, in the case where one wants to upgrade or modify the BrainPET system on the customer site, one does not have to replace the insert but instead only replaces the amplifier board. This is important because at the customer installation, the insert is always in the MRI room, and so is in a more sensitive area, whereas the ADC system and amplifier boards may be in a room adjacent to the MRI room which are not in a sensitive area. This method provides a much easier and safer upgrade path for existing BrainPET installations.

A sensitive area may include MRI and PET systems that are used for imaging during surgical procedures. If the PET system is located within the surgical field then any change to the PET insert portion inside the bore would be in conflict with maintaining a higher level of sterility within the surgical field.

This approach leaves the insert and cable the same but changes only the amplifier board size and ADC size because the changes are not made in the insert, but instead made in the ADC and amplifier area, as discussed herein.

In some embodiments, the block select line is generated inside the ADC electronics cabinet.

As will be apparent to those of skill in the art, this method allows for the system to be upgraded without requiring an upgrade of the insert, as discussed herein.

Specifically, the advantage of this approach is that the amount of muxing can be chosen after the insert installation is done, either 2:1, 4:1 or more, based on the type of noise-equivalent count rate (NECR) curve that the customer wants to achieve. In the prior art example above, the insert was designed with 4 detector blocks being read-out using 8 output lines. All 4 detector blocks had their outputs A, B, C and D joined together, which can be represented by 4:1 meaning 4 blocks are reduced to 1 set of output lines.

Alternatively, for some applications it might be better to only have 2 detector blocks multiplexed together, which can be called 2:1 muxing. In the prior art method, the insert is designed in advance to only allow 4:1 muxing. In this new method, one can more easily upgrade or modify the muxing to be either 2:1 or 4:1 or even 8:1 after the fact by simply modifying the electronics cabinet, as discussed herein.

For example, in some cases, customers want to have an insert which is capable of use in a maximum design, but they want to choose different electronic cabinets for different applications. For example, assume that a customer has 2 MRI systems. The first system is used for research, and in this system a maximum design is required in order to provide the highest count rate possible. In the second MRI, the applications are clinical imaging at a lower count rate requirement. In this example, assume that the insert can be easily moved between these two MRI systems, but the electronics cabinets are not easy to move due to specific restrictions at the clinical site. The customer can move only the insert and can select the electronic cabinet that suits the application of the first and second MRI, so that the customer can save money by using one insert system shared between the clinical and research groups. In this case, the insert would be a maximum capable insert, the electronics cabinet sold to the research group would allow the maximum amount of count rate, but the electronics cabinet sold to the clinical group would be a reduced cost cabinet that allows 2:1 or 4:1 or 8:1 multiplexing. In the prior art method, this usage of a single insert for both maximum 1:1 and cost reduced 2:1, 4:1 or 8:1 count rates is not possible.

While the preferred embodiments of the invention have been described above, it will be recognized and understood that various modifications may be made therein, and the appended claims are intended to cover all such modifications which may fall within the spirit and scope of the invention.

## Claims

1. A positron emission tomography imaging system comprising:
a plurality of slow output cables, each slow output cable having a detector end and at least a first chip end;
a plurality of detector blocks, each respective corner of each one of the plurality of the detector blocks being connected to the detector end of a respective one of the plurality of slow output cables;
at least a first respective one detector block and a second respective one detector block being multiplexed together by connecting the first chip end of the slow output cable connected to the first respective one detector block and the first chip end of the slow output cable connected to the second respective one detector block to one analog-to-digital converter chip.

2. The positron emission tomography imaging system according to claim 1 wherein the first respective one detector block and the second respective one detector block are geometrically positioned with the positron emission tomography imaging system such that the first respective one detector block and the second respective one detector block cannot be in coincidence.

3. The positron emission tomography imaging system according to claim 1 further comprising a plurality of fast output cables, each respective one fast output cable connected at a detector end to a respective one detector block and at a first chip end to a timing chip.

4. The positron emission tomography imaging system according to claim 3 wherein each fast output cable has a second chip end connected to a block selection chip.

5. The positron emission tomography imaging system according to claim 1 wherein each slow output cable has a second chip end for connecting each corner of a given detector block to one analog-to-digital converter chip, one timing chip or one block selection chip.

6. The positron emission tomography imaging system according to claim 4 wherein each slow output cable has a third chip end for connecting each corner of a given detector block to a timing chip.
